# EUROPÄISCHE PATENTANMELDUNG

(11) **EP 2 550 945 A1**
(43) Veröffentlichungstag der Anmeldung: **30.01.2013**
(21) Anmeldenummer: 11175780.3
(22) Anmeldetag: 28.07.2011
(51) Int. Cl.: A61F 13/20, A61F 13/34, A61K 9/00, A61H 21/00

(54) **Tampon mit einem Hohlraum**

(71) Anmelder: Marlafin AG, 6332 Hagendorn (CH)
(72) Erfinder: Hammen, Axel, 5426 Lengnau (CH); Lopez, Odilo, 8224 Löhningen (CH)
(74) Vertreter: AMMANN PATENTANWÄLTE AG BERN

(57) **Zusammenfassung**

Die Erfindung löst die Aufgabe, einen Tampon (1) mit einem Hohlraum zur Aufnahme einer Kapsel zur Verfügung zu stellen. Die Kapsel kann beispielsweise ein Vibrationselement enthalten. Ser Hohlraum ist durch einen auf mindestens einer Seite offenen Hohlkörper (5) gebildet, der auf mindestens einem Teil seiner Länge vom saugfähigen Material (2) umgeben, vorzugsweise umwickelt ist.

## Beschreibung

Die Erfindung betrifft einen Tampon aus saugfähigem Material mit einem Hohlraum zur Aufnahme einer Kapsel.

Aus den Dokumenten US5782779, US6183428 und US2007/0260210 sind Tamponanordnungen mit einem im Tamponkörper eingebetteten Vibrationselement bekannt. Solche Tamponanordnungen werden gegen Menstruationsbeschwerden eingesetzt. Was die Einbettung des Vibrationselements in den Tamponkörper anbelangt, kann lediglich dem Dokument US2007/0260210 entnommen werden, dass das Vibrationselement in einer Kapsel enthalten ist, an deren Aussenseite Rückhalteelemente angeordnet sind, um zu verhindern, dass die Kapsel insbesondere beim Zurückziehen der Anordnung aus der Vagina aus dem Tamponkörper herausgerissen wird. Der Tamponkörper ist in diesem Dokument als saugfähiger Körper mit einem Hohlraum beschrieben, in den die Kapsel offenbar hineingesteckt wird. Der Aufbau dieses Tamponkörpers oder dessen Herstellung ist aber in den genannten Dokumenten nicht beschrieben.

Ausgehend von diesem Stand der Technik liegt der Erfindung die Aufgabe zugrunde, einen Tampon mit einem Hohlraum zur Aufnahme einer Kapsel anzugeben, der einfach und kostengünstig herstellbar ist. Der Ausdruck Kapsel soll im Zusammenhang mit der Erfindung nicht auf ein Vibrationselement beschränkt werden.

Diese Aufgabe wird erfindungsgemäss dadurch gelöst, dass der Hohlraum durch einen auf mindestens einer Seite offenen Hohlkörper gebildet ist, der auf mindestens einem Teil seiner Länge vom saugfähigen Material umgeben ist.

Diese Lösung hat insbesondere den Vorteil, dass der Hohlkörper vorgefertigt und mit dem saugfähigen Material bedeckt, beispielsweise umwickelt werden kann. Das saugfähige Material kann beispielsweise Fasermaterial sein, jedoch soll der Begriff im vorliegenden Zusammenhang jegliches Material umfassen, das fähig und geeignet ist, Flüssigkeit aufzusaugen, beispielsweise auch Material mit einer schaumartigen Struktur.

Nach einer Ausführungsart hat der Hohlkörper Rückhaltemittel für eine in seinem Inneren aufzunehmende Kapsel. Damit kann diese Kapsel sicher im Tampon verankert werden.

Eine weitere Ausführungsart sieht vor, dass die Rückhaltemittel als im Inneren des Hohlkörpers angeordnete, umlaufende Nut ausgebildet sind. Eine solche Nut ist einfach herzustellen und kann mindestens eine am Umfang der Kapsel vorgesehene Erhebung aufnehmen.

Nach anderen Ausführungsarten sind die Rückhaltemittel als mindestens eine Rastnase ausgebildet und die Rastnase ist am freien Ende einer Federzunge angeordnet. Die Rastnase kann am freien Ende einer Kapsel angreifen oder in eine am Umfang der Kapsel vorgesehene Vertiefung, beispielsweise eine Ringnut, eingreifen und damit die Kapsel im Tampon festhalten.

Gemäss einer weiteren Ausführungsart ist vorgesehen, dass der Hohlkörper mindestens ein im saugfähigen Material eingebettetes Verankerungselement aufweist. Damit wird zuverlässig verhindert, dass der Hohlkörper aus dem saugfähigen Material herausgezogen wird.

Eine weitere Ausführungsart sieht vor, dass das Verankerungselement länglich ausgebildet ist, mindestens annähernd parallel zur Längsachse des Hohlkörpers orientiert ist und in mindestens einem Endbereich des Hohlkörpers mit diesem verbunden ist. Diese Ausgestaltung erlaubt es, ein Band aus saugfähigem Material zwischen dem Umfang des Hohlkörpers und dem Verankerungselement festzuhalten und anschliessend das saugfähige Material um den Hohlkörper und das Verankerungselement herumzuwickeln, um einen Tampon zu bilden.

Gemäss einer anderen Ausführungsart weist der Hohlkörper eine durchbrochene Mantelfläche auf. Diese Ausbildung in der Art eines Korbes erlaubt es, bei gegebenen Aussenabmessungen mehr saugfähiges Material einzubringen als beispielsweise mit einer Hülse mit geschlossener Wandung.

Nach einer besonderen Ausführungsart ist der Hohlkörper zumindest an seiner inneren Oberfläche mit einer antimikrobiellen Substanz ausgestattet. Damit wird insbesondere Infektionen vorgebeugt, falls eine im Tampon verwendete Kapsel ungenügend gereinigt wurde. Die antimikrobielle Substanz kann dabei beispielsweise Silber sein oder enthalten und sie kann als Schicht auf dem Hohlkörper aufgebracht oder als Partikel, beispielsweise Nanopartikel, im Material des Hohlkörpers aufgenommen sein.

Ein anderer Aspekt der Erfindung betrifft eine Vorrichtung zum Entfernen einer in einem Tampon angeordneten Kapsel. Diese Vorrichtung weist ein Element mit einer Öffnung auf, deren lichte Weite höchstens der lichten Weite des Hohlkörpers entspricht.

Diese Merkmale haben den Vorteil, dass eine in einem Tampon aufgenommene Kapsel durch die Öffnung hindurch herausgezogen werden kann, während der Tampon durch das Element zurückgehalten wird.

Nach einer Ausführungsart der Vorrichtung weist das Element einen von der Öffnung ausgehenden, radialen, offenen Schlitz auf. Dies hat bei der Verwendung einer Kapsel mit einem Zugelement, beispielsweise eines Rückzugsfadens oder eines Kabels, den Vorteil, dass das Zugelement in diesen Schlitz gelegt werden kann.

Gemäss einer weiteren Ausführungsart weist das Element Freigabemittel zum Bewegen von als Rastnasen ausgebildeten Rückhaltemitteln auf. Dies erlaubt eine leichte und zuverlässige Entfernung einer im Hohlraum des Tampons angeordneten Kapsel. Bevorzugt sind diese Freigabemittel als konische Hülse ausgebildet. Durch diese werden die Rastnasen nach aussen gedrängt, wenn der Tampon gegen die konische Hülse bewegt wird.

Eine weitere Ausführungsart sieht vor, dass die Vorrichtung ein Behältnis zur Aufnahme des Tampons aufweist. Dadurch muss der Tampon zum Entfernen der Kapsel nicht angefasst werden und kann mitsamt dem Behältnis entsorgt werden.

Entsprechend einer weiteren Ausführungsart besteht das Behältnis aus einer einseitig geschlossenen Hülse. Ein solches Behältnis ist besonders einfach herstellbar. Schliesslich ist nach einer anderen Ausführungsart vorgesehen, dass die Hülse in Längsrichtung geteilt ist und aus zwei Halbschalen besteht, die vorzugsweise durch ein Scharnier miteinander verbunden sind. Ein solches Behältnis ist besonders einfach in der Handhabung.

Ausführungsbeispiele der Erfindung werden nachstehend unter Bezugnahme auf die angefügten Zeichnungen beispielsweise näher beschrieben. Es zeigt
- Figur 1: eine teilweise aufgeschnittene perspektivische Ansicht eines Tampons,
- Figur 2: eine perspektivische Ansicht eines Hohlkörpers,
- Figur 3: in einem Längsschnitt einen Ausschnitt einer anderen Ausführungsart eines Tampons mit im Hohlkörper eingesetzter Kapsel mit einem Rückzugselement,
- Figur 4: eine perspektivische Ansicht einer Vorrichtung zum Entfernen der Kapsel und
- Figuren 5a und 5b: eine Schnittansicht einer weiteren Vorrichtung zum Entfernen der Kapsel.

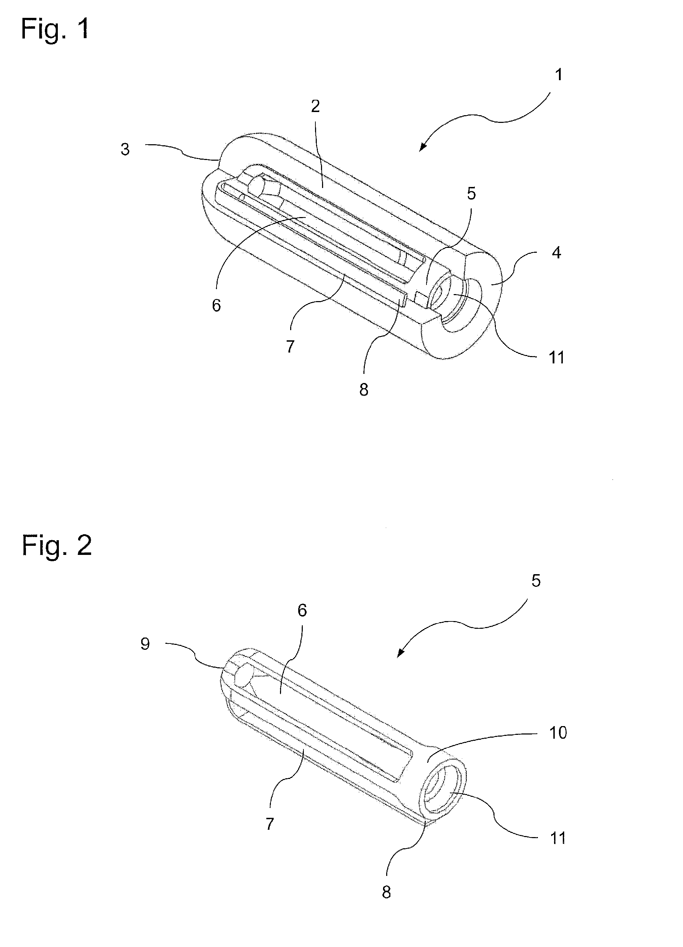

Figur 1 zeigt einen Tampon 1 in einer perspektivischen Ansicht. Er besteht aus saugfähigem Material 2 und hat ein vorderes, abgerundetes Ende 3 und ein hinteres Ende 4. Der Tampon 1 muss nicht wie dargestellt zylindrisch ausgebildet sein, sondern kann auch eine andere Längskontur und eine andere Querschnittsform aufweisen. Im Inneren des saugfähigen Materials 2 ist ein Hohlkörper 5 zu erkennen, der mit einem Verankerungselement 7 im saugfähigen Material 2 fixiert ist. Die Funktion des Verankerungselements 7 wird nachstehend im Zusammenhang mit Figur 2 noch näher erläutert.

Figur 2 zeigt den Hohlkörper 5 von Figur 1 ohne umgebendes saugfähiges Material 2. Der Hohlkörper 5 weist in seiner Wandung Durchbrechungen 6 auf, sodass man ihn auch als Korb bezeichnen kann, und hat ein vorderes Ende 9 und ein hinteres Ende 10. Der Hohlkörper besteht vorzugsweise aus einem Kunststoff und ist im Spritzgussverfahren hergestellt. Im Beispiel gemäss Figur 2 ist das hintere Ende 10 des Hohlkörpers 5 ringförmig ausgebildet und hat an seiner Innenfläche eine Ringnut 11, die als Rückhaltemittel für eine in den Hohlkörper 5 eingeführte Kapsel dient. Die Kapsel kann beispielsweise ein kapselförmiges Vibrationselement sein, das durch die Rückhaltemittel im Hohlkörper 5 fixiert wird. Die Kapsel kann zu diesem Zweck an ihrem Umfang mit einzelnen Erhebungen oder einer umlaufenden Rippe versehen sein, die in der Ringnut 11 einrastet. Ein Verankerungselement 7 ist am vorderen Ende 9 des Hohlkörpers 5 angeformt, hat ein freies Ende 8 und erstreckt sich etwa parallel zur Längsachse des Hohlkörpers 5. Bei der Herstellung des Tampons 1 wird ein Band aus einem saugfähigen Material zwischen das Verankerungselement 7 und den Mantel des Hohlkörpers 5 geführt, ähnlich wie bei einem Clip eines Schreibstiftes. Danach wird das saugfähige Material um den Hohlkörper 5 herumgewickelt, wobei es schliesslich auch das Verankerungselement 7 bedeckt. Als Variante kann das Verankerungselement 7 auch in Art einer Öse, also ohne freies Ende 8, ausgebildet sein. Der fertig gewickelte Tampon kann noch verdichtet werden, wie dies bei der Tamponherstellung üblich ist. Damit dabei der Hohlkörper 5 nicht beschädigt wird, kann dieser während des Verdichtungsvorgangs vorübergehend einen passenden Dorn aufnehmen. Bei der Verwendung eines korbartig durchbrochenen Hohlkörpers 5 lässt sich infolge der Durchbrechungen bei gegebenem Aussendurchmesser etwas mehr saugfähiges Material einbauen als bei einer geschlossenen Hülse. Ausserdem werden bei Verwendung eines Vibrators die von diesem ausgehenden Schwingungen durch die Durchbrechungen hindurch direkt an das saugfähige Material übertragen.

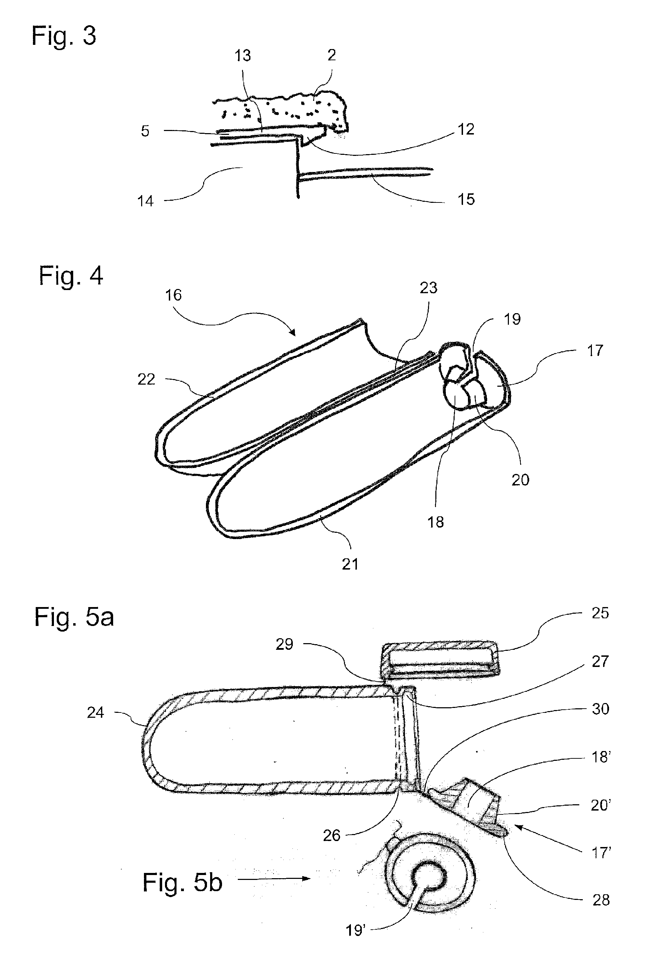

Figur 3 zeigt eine alternative Ausführungsart von Rückhaltemitteln, die hier als mindestens eine Rastnase 12 ausgebildet sind, die ihrerseits am freien Ende einer Federzunge 13 angeordnet ist. Mit 14 ist eine Kapsel bezeichnet, die durch die an ihrer rückwärtigen Stirnseite angreifende Rastnase 12 im Inneren des Hohlkörpers 5 festgehalten wird. An der Kapsel 14 ist ein Rückzugselement 15 befestigt, das dazu dient, eine aus dem Tampon 1 und der Kapsel 14 bestehende Einheit beispielsweise aus einer Körperöffnung herauszuziehen.

Figur 4 zeigt eine Vorrichtung 16 zum Entfernen einer Kapsel 14 aus einem Tampon 1 der vorangehend beschriebenen Art. Ein scheibenartiges Element 17 hat eine zentrale Öffnung 18, durch welche die Kapsel 14 passt. Ausserdem ist im Element 17 ein radialer Schlitz 19 vorhanden, der zur Aufnahme des erwähnten Rückzugselements 15 bestimmt und dimensioniert ist. Bei einem mit einem Hohlkörper gemäss Figur 2 ausgestatteten Tampon 1 wird die Kapsel 14 entfernt, indem das Rückzugselement 15 in den Schlitz 19 eingelegt, das Element 17 festgehalten und dann am Rückzugselement 15 gezogen wird. Dabei muss die Kombination aus der Ringnut 11 und der genannten Erhebungen bzw. der Rippe so gestaltet sein, dass die Kraft zum Herausziehen der Kapsel 14 aus dem Tampon 1 erheblich grösser ist als die erforderliche Kraft zum Zurückziehen des Tampons aus einer Körperöffnung. In einer vorteilhaften Weiterbildung hat das Element 17 einen Konus 20, der dazu bestimmt ist, die Rastnase 12 gemäss Figur 3 so zu bewegen, dass sie die Kapsel 14 freigibt, wenn der Tampon durch Ziehen am Rückzugselement 15 gegen den Konus 20 bewegt wird.

Damit der Tampon 1 beim Entfernen der Kapsel 14 nicht mit der Hand berührt werden muss, ist die Vorrichtung vorzugsweise mit einem Aufnahmebehältnis ausgestattet. Im Beispiel gemäss Figur 4 besteht das Aufnahmebehältnis aus zwei Halbschalen 21, 22, die durch ein Filmscharnier 23 miteinander verbunden sind und das Element 17 ist an einer 21 der Halbschalen befestigt. Zum Entfernen der Kapsel 14 wird der Tampon 1 am Rückzugselement festgehalten und in die Halbschale 21 gelegt und das Rückzugselement 15 in den Schlitz 19 eingeführt. Danach wird am Rückzugselement 15 gezogen, sodass die Rastnasen 12 mit dem Konus 20 in Kontakt treten und in der Folge nach aussen gedrängt werden und dadurch die Kapsel 14 freigeben. Diese kann nun durch die Öffnung 18 herausgezogen werden. Vor oder nach dem Ziehen am Rückzugselement 15 können die Halbschalen geschlossen werden und der gebrauchte Tampon 1 kann nach dem Entfernen der Kapsel 14 mitsamt der Vorrichtung 16 entsorgt werden.

Die Figuren 5a und 5b zeigen eine alternative Ausführungsart der Vorrichtung, bei der das Aufnahmebehältnis als einseitig geschlossene Hülse 24 ausgebildet ist. Das Element 17' ist mittels eines Filmscharniers 30 mit der Hülse 24 verbunden, sodass es nach dem Einführen eines gebrauchten Tampons in die Hülse in deren offenes Ende eingesetzt werden kann, wo es durch Einrasten des Randes 28 in einer Ringnut 27 festgehalten wird. Diese Vorrichtung ist zusätzlich mit einem Deckel 25 ausgestattet, der durch ein Filmscharnier 29 mit der Hülse 24 verbunden sein kann und der nach dem Entfernen der Kapsel 14 auf das offene Ende der Hülse 24 gestülpt werden kann, wo er in einer äusseren Ringnut 26 einrastet. Damit ist das Behältnis zur Entsorgung dicht verschlossen. Ein entsprechender Deckel kann auch beim Beispiel nach Figur 4 vorgesehen werden. Figur 5b zeigt in einer Draufsicht auf das Element 17', dass auch dieses einen Schlitz 19' zur Aufnahme des Rückzugselements 15 hat. Die Halbschalen 21, 22 oder die Hülse 24 können beispielsweise aus einem Kunststoff oder aus Karton bestehen.

### Bezugszeichenliste

- 1: Tampon
- 2: saugfähiges Material
- 3: vorderes Ende
- 4: hinteres Ende
- 5: Hohlkörper
- 6: Durchbrechungen
- 7: Verankerungselement
- 8: freies Ende von 7
- 9: vorderes Ende
- 10: hinteres Ende
- 11: Ringnut
- 12: Rastnase
- 13: Federzunge
- 14: Kapsel
- 15: Rückzugselement
- 16: Vorrichtung
- 17,: 17' Element
- 18,: 18' Öffnung
- 19,: 19' Schlitz
- 20,: 20' Konus
- 21: Halbschale
- 22: Halbschale
- 23: Filmscharnier
- 24: Hülse
- 25: Deckel
- 26: Ringnut aussen
- 27: Ringnut innen
- 28: Rand
- 29: Filmscharnier
- 30: Filmscharnier

## Patentansprüche

1. Tampon (1) aus saugfähigem Material (2) mit einem Hohlraum zur Aufnahme einer Kapsel (14), **dadurch gekennzeichnet, dass** der Hohlraum durch einen auf mindestens einer Seite offenen Hohlkörper (5) gebildet ist, der auf mindestens einem Teil seiner Länge vom saugfähigen Material (2) umgeben ist.

2. Tampon nach Anspruch 1, **dadurch gekennzeichnet, dass** der Hohlkörper (5) Rückhaltemittel (11, 12) für eine in seinem Inneren aufzunehmende Kapsel (14) hat.

3. Tampon nach Anspruch 2, **dadurch gekennzeichnet, dass** die Rückhaltemittel als im Inneren des Hohlkörpers (5) angeordnete, umlaufende Nut (11) ausgebildet sind.

4. Tampon nach Anspruch 2, **dadurch gekennzeichnet, dass** die Rückhaltemittel als mindestens eine Rastnase (12) ausgebildet sind.

5. Tampon nach Anspruch 4, **dadurch gekennzeichnet, dass** die Rastnase (12) am freien Ende einer Federzunge (13) angeordnet ist.

6. Tampon nach einem der vorangehenden Ansprüche, **dadurch gekennzeichnet, dass** der Hohlkörper (5) mindestens ein im saugfähigen Material eingebettetes Verankerungselement (7) aufweist.

7. Tampon nach Anspruch 6, **dadurch gekennzeichnet, dass** das Verankerungselement (7) länglich ausgebildet ist, mindestens annähernd parallel zur Längsachse des Hohlkörpers (5) orientiert ist und in mindestens einem Endbereich (9) des Hohlkörpers (5) mit diesem verbunden ist.

8. Tampon nach einem der vorangehenden Ansprüche, **dadurch gekennzeichnet, dass** der Hohlkörper (5) eine durchbrochene Mantelfläche aufweist.

9. Tampon nach einem der vorangehenden Ansprüche, **dadurch gekennzeichnet, dass** der Hohlkörper (5) zumindest an seiner inneren Oberfläche mit einer antimikrobiellen Substanz ausgestattet ist.

10. Vorrichtung (16) zum Entfernen einer in einem Tampon (1) nach Anspruch 2 angeordneten Kapsel (14), **dadurch gekennzeichnet, dass** die Vorrichtung (16) ein Element (17) mit einer Öffnung (18) aufweist, deren lichte Weite höchstens der lichten Weite des Hohlkörpers (5) entspricht.

11. Vorrichtung nach Anspruch 10, **dadurch gekennzeichnet, dass** das Element (17) einen von der Öffnung (18) ausgehenden, radialen, offenen Schlitz (19) aufweist.

12. Vorrichtung nach einem der Ansprüche 10 bis 11 zum Entfernen einer in einem Tampon (1) nach einem der Ansprüche 4 bis 5 angeordneten Kapsel, **dadurch gekennzeichnet, dass** das Element (17) Freigabemittel (20) zum Bewegen der Rastnasen (12) aufweist.

13. Vorrichtung nach Anspruch 12, **dadurch gekennzeichnet, dass** die Freigabemittel als konische Hülse (20) ausgebildet sind.

14. Vorrichtung nach einem der Ansprüche 10 bis 13, **dadurch gekennzeichnet, dass** sie ein Behältnis (21, 22; 24) zur Aufnahme des Tampons (1) aufweist.

15. Vorrichtung nach Anspruch 14, **dadurch gekennzeichnet, dass** das Behältnis aus einer einseitig geschlossenen Hülse besteht.

16. Vorrichtung nach Anspruch 15, **dadurch gekennzeichnet, dass** die Hülse in Längsrichtung geteilt ist und aus zwei Halbschalen (21, 22) besteht, die vorzugsweise durch ein Scharnier (23) miteinander verbunden sind.
